# EUROPEAN PATENT SPECIFICATION

(11) **EP 3 176 578 B1**
(45) Date of publication and mention of the grant of the patent: **01.05.2024**
(21) Application number: 16201675.2
(22) Date of filing: 01.12.2016
(51) Int. Cl.: G01N 33/00, G01N 33/18, G01N 1/22, G01N 1/34

(54) **PREPROCESSING APPARATUS AND METHOD FOR GAS ANALYSIS**
VORBEARBEITUNGSVORRICHTUNG UND -VERFAHREN ZUR GASANALYSE
APPAREIL ET PROCÉDÉ DE PRÉTRAITEMENT POUR L'ANALYSE DE GAZ

(30) Priority: 01.12.2015 JP 2015234839; 08.11.2016 JP 2016217844
(43) Date of publication of application: 07.06.2017
(73) Proprietor: Japan Agency for Marine-Earth Science and Technology, Yokosuka-shi Kanagawa 237-0061 (JP)
(72) Inventor: SATO, Keiko, Yokosuka-shi, Kanagawa 237-0061 (JP); KUMAGAI, Hidenori, Yokosuka-shi, Kanagawa 237-0061 (JP)
(74) Representative: Paulraj, Leonita Theresa

(56) References cited:
- WO-A1-97/46755
- US-A- 3 545 929
- US-A1- 2002 024 662
- YUJI SANO ET AL: "Measurement of Noble Gas Solubility in Seawater Using a Quadrupole Mass Spectrometer", JOURNAL OF OCEANOGRAPHY ; EDITED BY THE OCEANOGRAPHIC SOCIETY OFJAPAN, KLUWER ACADEMIC PUBLISHERS-CONSULTANTS BUREAU, NE, vol. 61, no. 3, 1 June 2005 (2005-06-01), pages 465-473, XP019249880, ISSN: 1573-868X

## Description

### TECHNICAL FIELD

The present invention relates to a preprocessing apparatus and a preprocessing method for gas analysis for use before a gas to be analyzed is introduced into a gas analysis device.

### BACKGROUND ART

Seafloor hydrothermal deposits have been eagerly explored and developed, and it is an urgent matter to establish a method of exploring the seafloor hydrothermal deposits. It is considered, as one exploration method, to measure a rare gas dissolved in seawater in order to track hydrothermal plumes advected and diffused in the seawater. For the purpose of this exploration, it is necessary to separate and extract the rare gas, as a target gas to be extracted, from a sampled aqueous solution.

For example, JP 5461144 B2 (Patent Document 1) and JP H7-33417 A (Patent Document 2) disclose a technique of removing impurities from a high-pressure rare gas.

A reference titled "Measurement of Noble Gas Solubility in Seawater Using a Quadrupole Mass Spectrometer," written by SANO Yuji and TAKAHATA Naoto, Journal of Oceanography, Vol. 61, pp. 465 to 473, 2005 (Non-Patent Document 1) discloses a method of separating and extracting a rare gas contained in seawater.
US 2002/024662 relates to a micro-fluidic cell for optical detection of gases compirsing a concentration cell and a detection cell, and increases the sensitivity of optical detection of gases, selectivity of components, and accuracy of quantitative determination, and also achieves a low electric power consumption and a small-sized, light-weight configuration of the entire apparatus.
WO 97/46755 relates to a method of predicting, during the production of paper or paperboard, the concentration of one or several particular volatile organic compound(s)
(VOC) which is (are) emitted by the finished pulp, paper or paperboard product, is described. In the method the concentration of one or several particular VOC is determined in the gas/vapor phase emitted by the pulp, paper or paperboard web during production. The determination can be performed with the aid of infrared spectroscopy or thermal description gas chromatography during the production, and the obtained results of the measurement can be compared with the reference values obtained by analyses of the finished pulp, paper or paperboard product for calibration of the method.
US 3545929 patented on 08 December 1970 relates to a method measuring trace amounts of carbon monoxide dissolved in water along with light hydrocarbons including methane.

### SUMMARY OF INVENTION

### TECHNICAL PROBLEM

The techniques described in Patent Document 1 and Patent Document 2 are intended to increase the purity of a high-pressure rare gas for use in a semiconductor manufacturing process, and may not be directly applied to the separation and extraction of a minute (trace) amount of a rare gas contained in a sample.

The method described in Non-Patent Document 1 is directed to seawater. Seafloor hydrothermal fluid has properties of both hot spring water and seawater. Therefore, it is expected that large amounts of a halogen compound and a sulfur compound are dissolved in seafloor hydrothermal fluid, and the halogen compound and the sulfur compound that are dissolved in large amounts may not completely be removed by the method of Non-Patent Document 1. Therefore, the halogen compound may adversely affect the analysis and the sulfur compound may damage the analysis device and a flow path through which a sample passes. Thus, it is necessary to precisely remove the halogen compound and the sulfur compound. In particular, if the analysis device and the flow path through which a sample passes are all constituted of metal in order to purify and measure a rare gas, the analysis device and the flow path tend to be corroded by the effect of the halogen compound and the sulfur compound. Therefore, corrosion can be significantly prevented by precisely removing the halogen compound and the sulfur compound.

Further, the method described in Non-Patent Document 1 is based on the "spike method" in which a measurement is performed with the addition of a substance called a "spike" which has an isotope ratio different from that of a sample and the ratio and the amount of which are known (in Non-Patent Document 1, specifically, a mixture of ²²Ne, ³⁶Ar, ⁸⁶Kr, and ¹²⁴Xe). Therefore, in a water trapping section, seawater as a sample is rapidly cooled using a refrigerant (at about -80°C) of ethanol and dry ice to remove a water content (page 468, left column, lines 2 to 4). Therefore, a target gas which should not be removed may be partially removed together with the water. Thus, the method described in Non-Patent Document 1 is not suitable for analysis in which a minute (trace) amount of a rare gas is separated and extracted without using the spike method.

It is an object of the present invention to provide a preprocessing apparatus and a preprocessing method for gas analysis that are capable of separating and extracting a rare gas (target gas to be extracted) as a target gas with high purity from sample water, such as seafloor hydrothermal fluid, in which a halogen compound, a sulfur compound, and the rare gas are dissolved.

It is another object of the present invention to provide a preprocessing apparatus and a preprocessing method for gas analysis that use a getter capable of precisely removing a halogen compound and a sulfur compound.

### SOLUTION TO PROBLEM

In one aspect of the present invention, there is provided a preprocessing apparatus for gas analysis that includes, as basic components, a water trapping section and an adsorption section. The water trapping section is configured to trap water in sample water, in which a target gas to be extracted is dissolved, to obtain a to-be-purified target gas (a target gas to be purified). The adsorption section is configured to adsorb a non-target gas for extraction in the to-be-purified target gas.

The water trapping section includes a first cold trap and a second cold trap. The first cold trap is configured to cool the sample water, which has flowed in a first trap path, at a first temperature at which the target gas is not condensed, thereby removing a liquid phase to extract to-be-purified target gas containing water vapor, and to establish a first vacuum environment in the first trap path. The second cold trap is configured to further cool the to-be-purified target gas containing water vapor, which has been purified (dried) by the first cold trap and then has flowed in a second trap path, at a second temperature at which the water vapor is condensed but the target gas is not condensed and which is lower than the first temperature, thereby removing the water vapor to obtain the to-be-purified target gas not containing water vapor, and to establish a second vacuum environment in the second trap path such that the pressure in the second vacuum environment is lower than that in the first vacuum environment. The adsorption section includes one or more getters operable to adsorb the non-target gas in the to-be-purified target gas, which has flowed in a getter placement path, and to establish a vacuum environment in the getter placement path such that the pressure in the getter placement path is lower than that in the water trapping section.

The wording "not containing" or "does not contain" as used herein is used in the broad sense, and means not only a state in which something is not contained at all (0%) but also a state in which something is not substantially contained. For example, the " to-be-purified target gas not containing water vapor" refers to a to-be-purified target gas from which water vapor has been removed by the second cold trap to such a degree that water vapor is not detected when the analysis device measures the target gas.

In the present invention, as described above, the water trapping section is divided into the first cold trap and the second cold trap to trap water and water vapor stepwise. By processing the sample water in this way, it is possible to prevent blockage of a flow path due to rapid growth of ice, and also to prevent the target gas as the subject substance from being removed together with water and water vapor. Then, the adsorption section adsorbs the non-target gas. Thus, the target gas with high purity can be fed to the analysis device. This improves accuracy (in particular, quantitative or metering accuracy in isotope analysis).

One or more getters are provided in the adsorption section. How to configure the getter depends on the kind of the non-target gas for extraction. For example, if the non-target gas is the halogen compound and the sulfur compound dissolved in the sample water, the adsorption section may be configured to include a first adsorption sub-section provided with a first getter and a second adsorption sub-section provided with a second getter. The first getter adsorbs a halogen compound in the to-be-purified target gas. The second getter adsorbs a sulfur compound in the to-be-purified target gas which has been subjected to an adsorption process performed by the first adsorption sub-section.

In the preprocessing apparatus for gas analysis according to the present invention, the water trapping section may have any configuration, and the adsorption section includes one or more getters operable to adsorb the non-target gas in the to-be-purified target gas that has flowed in a getter placement path. The adsorption section may be configured to include a first adsorption sub-section provided with a first getter and a second adsorption sub-section provided with a second getter. The first getter adsorbs a halogen compound in the to-be-purified target gas. The second getter adsorbs a sulfur compound in the to-be-purified target gas that has been subjected to an adsorption process performed by the first adsorption sub-section.

By causing the to-be-purified target gas, from which water has been trapped, to sequentially react in the first adsorption sub-section and the second adsorption sub-section, the halogen compound and the sulfur compound, which are contaminants, can be precisely removed. This prevents damage to the flow path and the analysis device, and also improves the analysis.

The first getter may have any structure. However, a getter effect can be achieved if the first getter includes an iron (Fe) based getter material, a magnesium (Mg) based getter material, and a calcium (Ca) based getter material that are arranged along an inflow path of the first getter for the to-be-purified target gas. Particularly suitably, the iron based getter material is an iron wire; the magnesium based getter material is a magnesium ribbon; and the calcium based getter material is granular calcium. According to the study by the inventors, a high getter effect was achieved when the weight ratio of the calcium based getter material, the magnesium based getter material, and the iron based getter material was 1±10% : 1±10% : 0.03±10% (0.9-1.1 : 0.9-1.1 : 0.027-0.033). Similarly, the second getter may have any structure. However, a getter effect can be achieved if the second getter includes a copper oxide (CuO) based getter material, an iron oxide (FeO) based getter material, a first aluminum (Al) based getter material, a vanadium (V) based getter material, a zirconium (Zr) based getter material, and a second aluminum (Al) based getter material that are arranged along an inflow path of the second getter for the to-be-purified target gas. Particularly suitably, the first aluminum based getter material and the second aluminum based getter material are each an aluminum foil; the zirconium based getter material is plate-shaped zirconium; the vanadium based getter material is plate-shaped vanadium; the iron oxide based getter material is an iron oxide wire; and the copper oxide based getter material is a copper oxide wire. According to the study by the inventors, a high getter effect was achieved when the weight ratio of the first aluminum based getter material, the zirconium based getter material, the vanadium based getter material, the second aluminum based getter material, the iron oxide based getter material, and the copper oxide based getter material was 0.5±10% : 1±10% : 1±10% : 0.5±10% : 1±10% : 2±10% (0.45-0.55 : 0.9-1.1 : 0.9-1.1 : 0.45-0.55 : 0.9-1.1 : 1.8-2.2).

The adsorption section may further include a third adsorption sub-section configured to adsorb an active gas in the to-be-purified target gas that has been subjected to an adsorption process performed by the second adsorption sub-section. The adsorption section may further include a fourth adsorption sub-section configured to adsorb hydrogen in the to-be-purified target gas that has been subjected to an adsorption process performed by the third adsorption sub-section. By configuring the adsorption section in this way, the purity of the target gas can be further enhanced.

The preprocessing apparatus for gas analysis according to the present invention may be directed to any sample water, and particularly suitably to seawater, seafloor hydrothermal fluid, on-shore hot spring water, or the like.

In another aspect of the present invention, there is provided a preprocessing method for gas analysis including the steps of trapping water in sample water, in which a target gas to be extracted is dissolved, to extract obtain a to-be-purified target gas and adsorb a non-target gas for extraction in the to-be-purified target gas. The step of trapping water includes a first trapping step and a second trapping step. The first trapping step is configured to cool the sample water, which has flowed in a first trap path, at a first temperature at which the target gas is not condensed, thereby removing a liquid phase to obtain the to-be-purified target gas containing water vapor, and to establish a first vacuum environment in the first trap path. The second trapping step is configured to further cool the to-be-purified target gas not containing water vapor, which has been purified (dried) in the first trapping step and then has flowed in a second trap path, at a second temperature at which the water vapor is condensed but the target gas is not condensed and which is lower than the first temperature, thereby removing the water vapor to obtain the to-be-purified target gas which does not contain water vapor, and to establish a second vacuum environment in the second trap path such that the pressure in the second vacuum environment is lower than that in the first vacuum environment. In this case, the adsorbing step is configured to use one or more getters to adsorb the non-target gas in the to-be-purified target gas that does not contain the water vapor and has flowed in a getter placement path, and to establish a vacuum environment in the getter placement path such that the pressure in the getter placement path is lower than that in the vacuum environment established in the step of trapping water.

In the preprocessing method for gas analysis according to the present invention, the water trapping step may have any configuration, and the adsorbing step use one or more getters to adsorb the non-target gas in the to-be-purified target gas, which has flowed in a getter placement path, and to establish a vacuum environment in the getter placement path such that the pressure in the getter placement pat is lower than that in the vacuum environment established in the step of trapping water. The adsorbing step may be configured to include a first adsorbing step of adsorbing a halogen compound in the to-be-purified target gas, and a second adsorbing step of adsorbing a sulfur compound in the to-be-purified target gas that has been subjected to an adsorption process performed in the first adsorbing step.

### BRIEF DESCRIPTION OF DRAWINGS

Fig. 1 is a schematic diagram illustrating an exemplary configuration of a preprocessing apparatus for gas analysis according to an embodiment of the present invention.
Fig. 2 is a schematic view illustrating the internal structure of a first getter.
Fig. 3 is a schematic view illustrating the internal structure of a second getter.
Fig. 4 is a flowchart illustrating a process in which a rare gas, as a target gas to be extracted, is separated and extracted using the preprocessing apparatus for gas analysis.
Figs. 5A and 5B illustrate a getter effect of a first getter.
Figs. 6A and 6B illustrate a getter effect of a second getter.
Fig. 7A illustrates the result of analyzing sample water that has not been subjected to a getter process. Fig. 7B illustrates the result of analyzing the sample water after a getter process was performed by the first getter. Fig. 7C illustrates the result of analyzing the sample water after a getter process was performed by the second getter.
Fig. 8A illustrates the result of analyzing sample water that has not been subjected to a getter process. Fig. 8B illustrates the result of analyzing the sample water after a getter process was performed by the second getter. Fig. 8C illustrates the result of analyzing the sample water after a getter process was performed by the first getter.

### DESCRIPTION OF EMBODIMENTS

A preprocessing apparatus and a preprocessing method for gas analysis according to an embodiment of the present invention will be described in detail below with reference to the accompanying drawings.

### <Overall Configuration>

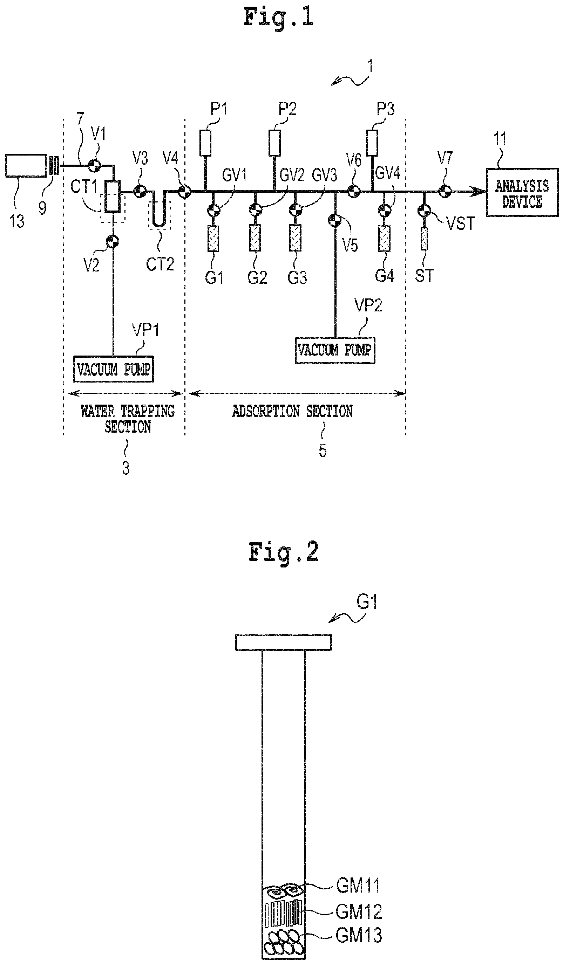

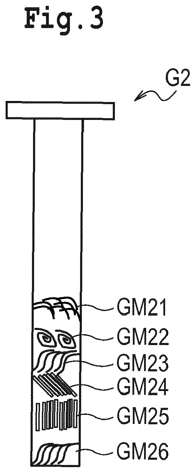

Fig. 1 is a schematic diagram illustrating the configuration of a preprocessing apparatus for gas analysis according to an embodiment. Fig. 2 illustrates the internal structure of a first getter G1. Fig. 3 illustrates the internal structure of a second getter G2.

A preprocessing apparatus 1 for gas analysis according to the embodiment is configured to separate and extract, from seafloor hydrothermal fluid as sample water, a rare gas dissolved in the seafloor hydrothermal fluid as a target gas to be extracted. In the seafloor hydrothermal fluid, rare gases, such as helium (He), neon (Ne), argon (Ar), krypton (Kr), and xenon (Xe) are dissolved. Halogen compounds, sulfur compounds, active gases (such as nitrogen, hydrocarbon, carbon monoxide, carbon dioxide, oxygen, hydrogen, and water vapor), and so forth are also dissolved therein. In the embodiment, the rare gases are target gases to be extracted, and the halogen compounds, the sulfur compounds, the active gases, and so forth are non-target gases for extraction.

The preprocessing apparatus 1 for gas analysis primarily includes a water trapping section 3 and an adsorption section 5. The water trapping section 3 is configured to trap water (a liquid phase and water vapor) in sample water, in which a target gas to be extracted is dissolved, to extract a to-be-purified target gas (a target gas to be purified). The adsorption section 5 is configured to adsorb a non-target gas for extraction in the to-be-purified target gas using a getter. The sample water introduced from a sample container connection port 9 of a conduit 7 passes through the conduit 7, and is processed by the water trapping section 3 and the adsorption section 5, and then is fed to an analysis device 11. The conduit 7 includes valves V1 to V7 configured to switch the flow path, and vacuum gauges P1 to P3 configured to measure the pressure in the conduit 7.

### [Water Trapping Section]

The water trapping section 3 includes a first cold trap CT1 and a second cold trap CT2. The first cold trap CT1 is configured to cool the sample water, which has flowed in a first trap path (a section of the conduit 7 delimited by the valves V1, V2, and V3), at a first temperature at which the target gas is not condensed, thereby removing a liquid phase, and to establish a first vacuum environment in the first trap path. In the embodiment, the first temperature is in the range of -10°C to -15°C (more preferably, -12°C to -15°C).

The second cold trap CT2 is configured to further cool the to-be-purified target gas containing water vapor, which has been purified (dried) by the first cold trap CT1 and then has flowed in a second trap path (a section of the conduit 7 delimited by the valves V3 and V4), at a second temperature at which the water vapor is condensed but the target gas is not condensed and which is lower than the first temperature, thereby removing the water vapor to obtain the to-be-purified target gas, and to establish a second vacuum environment in the second trap path such that the pressure in the second vacuum environment is lower than that in the first vacuum environment. In the embodiment, the second temperature is in the range of -25°C to -40°C (more preferably, -30°C to -35°C).

### [Adsorption Section]

The adsorption section 5 includes one or more getters configured to adsorb the non-target gas in the to-be-purified target gas not containing water vapor, which has flowed in a getter placement path (a section of the conduit 7 delimited by the valves V4 and V7), and to establish a third vacuum environment in the getter placement path such that the pressure in the getter placement path is lower than that in the second vacuum environment. Specifically, the adsorption section 5 includes a first adsorption sub-section provided with a first getter G1, a second adsorption sub-section provided with a second getter G2, a third adsorption sub-section provided with a third getter G3, and a fourth adsorption sub-section provided with a fourth getter G4.

The first getter G1 is configured to adsorb a halogen compound. As illustrated in Fig. 2, the first getter G1 includes an iron (Fe) based getter material GM11, a magnesium (Mg) based getter material GM12, and a calcium (Ca) based getter material GM13 that are arranged along an inflow path of the first getter G1 for the to-be-purified target gas. Specifically, the iron based getter material is an iron wire; the magnesium based getter material is a magnesium ribbon; and the calcium based getter material is granular calcium. According to an experiment conducted by the inventors, a high getter effect was achieved when the weight ratio of the calcium based getter material, the magnesium based getter material, and the iron based getter material was 1±10% : 1±10% : 0.03±10% (0.9-1.1 : 0.9-1.1 : 0.027-0.033).

As a matter of course, other materials may be used as the getter materials of the first getter G1. For example, a getter material including any one or any combination of nickel (Ni), cobalt (Co), copper (Cu), zirconium (Zr), and vanadium (V) may be used in place of or in addition to one or some or all of the getter materials described above.

The second getter G2 is configured to adsorb a sulfur compound (and an active gas generated when a sulfur compound is decomposed to be adsorbed). As illustrated in Fig. 3, the second getter G2 includes a copper oxide (CuO) based getter material GM21, an iron oxide (FeO) based getter material GM22, a first aluminum (Al) based getter material GM23, a vanadium (V) based getter material GM24, a zirconium (Zr) based getter material GM25, and a second aluminum (Al) based getter material GM26 that are arranged along an inflow path of the second getter G2 for the to-be-purified target gas. Specifically, the first aluminum based getter material and the second aluminum based getter material are each an aluminum foil; the zirconium based getter material is plate-shaped zirconium; the vanadium based getter material is plate-shaped vanadium; the iron oxide based getter material is an iron oxide wire; and the copper oxide based getter material is a copper oxide wire. According to an experiment conducted by the inventors, a high getter effect was achieved when the weight ratio of the first aluminum based getter material, the zirconium based getter material, the vanadium based getter material, the second aluminum based getter material, the iron oxide based getter material, and the copper oxide based getter material was 0.5±10% : 1±10% : 1±10% : 0.5±10% : 1±10% : 2±10% (0.45-0.55 : 0.9-1.1 : 0.9-1.1 : 0.45-0.55 : 0.9-1.1 : 1.8-2.2).

As a matter of course, other materials may be used as the getter materials of the second getter G2. For example, a getter material including any one or any combination of silver (Ag), gold (Au), platinum (Pt), and molybdenum (Mo) may be used in place of or in addition to either or both of the iron oxide based getter material and the copper oxide based getter material, among the getter materials described above. In addition, a getter material including any one or any combination of chromium (Cr), nickel (Ni), and cobalt (Co) may be used in place of or in addition to one or some or all of the zirconium based getter material, the vanadium based getter material, and the aluminum based getter materials.

The third getter G3 is configured to adsorb an active gas in the to-be-purified target gas which has been subjected to an adsorption process performed by the second adsorption sub-section. The third getter G3 includes a titanium (Ti) based getter material and a zirconium (Zr) based getter material that are arranged along an inflow path of the third getter G3 for the to-be-purified target gas.

The fourth getter G4 is configured to adsorb hydrogen. In the embodiment, a getter pump available from SAES getters S.p.A. under the name of SORB-AC (registered trademark) is used as the fourth getter G4.

### <Process Flow Until Target Gas is Introduced into Analysis Device>

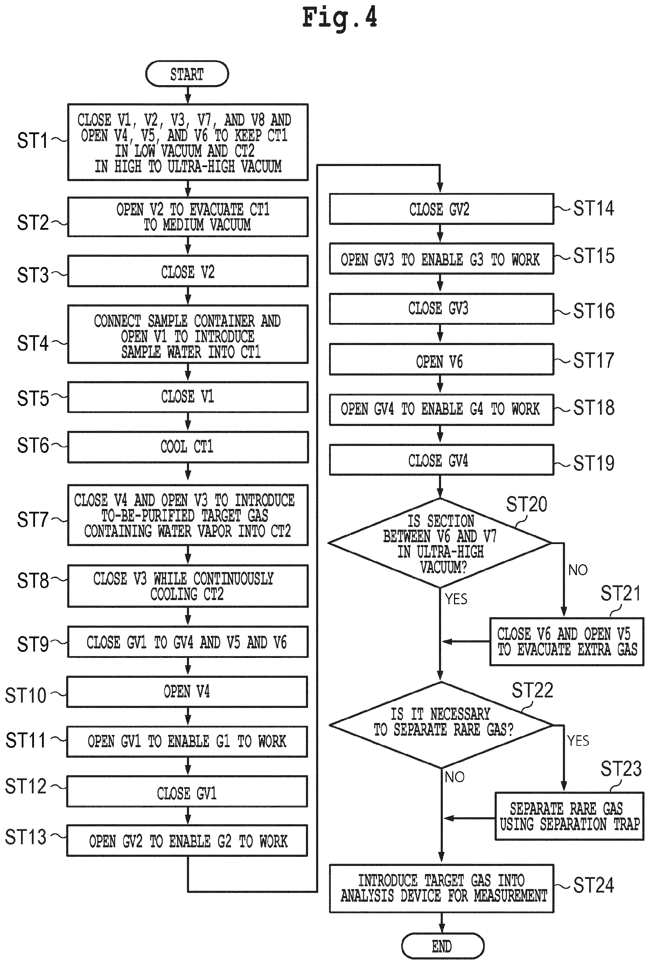

Fig. 4 is a flowchart of a process until a rare gas as a target gas to be extracted is introduced into the analysis device 11.

As described above, prior to the operation, the conduit 7 of the preprocessing apparatus 1 for gas analysis is pumped by a vacuum pump VP2 with the valves V1, V2, V3, and V7 closed and with the valves V4, V5, and V6 opened (step ST1). Consequently, prior to the operation, the inside of the second cold trap CT2 is kept in a vacuum environment corresponding to the high vacuum region to the ultra-high vacuum region. The inside of the first cold trap CT1 has been kept in a vacuum environment corresponding to the low vacuum region by an evacuation performed when the preceding analysis is ended.

First, a water trapping step is performed. In order to introduce a target gas into the analysis device 11, the valve V2 is first opened to evacuate the first cold trap CT1 to a pressure in the medium vacuum region (step ST2), and then the valve V2 is closed (step ST3). As needed, the degree of vacuum is checked using the vacuum gauge P1 by closing getter valves GV1 to GV3 and the valve V6 (and the valve V5 when it is opened) and opening the valve V3. As long as a vacuum pump VP1 is operating, a pressure in the medium vacuum region is readily reached by evacuation for about five minutes. Next, a sample container 13 in which sample water is stored is connected to the sample container connection port 9. The valve V1 is opened to introduce the sample water into the first cold trap CT1 (step ST4), and the valve V1 is quickly closed (step ST5). In the embodiment, the sample water is seafloor hydrothermal fluid and is stored in the sample container 13. When the valve V1 is opened and the sample water enters the first cold trap CT1, a pressure in the first cold trap CT1 is raised to the low vacuum region. In this state, the first cold trap CT1 is cooled at a first temperature, thereby removing a liquid phase (step ST6). Consequently, a pressure in the first cold trap CT1 decreases to become lower than that before cooling. Next, the valve V4 is closed and the valve V3 is opened to introduce a to-be-purified target gas containing water vapor into the second cold trap CT2 (step ST7). Consequently, a pressure in the second cold trap CT2 is raised around the boundary between the low vacuum region and the medium vacuum region. In this state, the second cold trap CT2 is cooled at a second temperature, thereby causing water vapor condensed. The valve V3 is closed while continuing to cool the second cold trap CT2 (step ST8). Consequently, a vacuum environment in the second cold trap CT2 decreases to the medium vacuum region.

Next, an adsorbing step is performed. First, the getter valves GV1 to GV4, which are attached to the first to fourth getters, and the valves V5 and V6 are closed (step ST9). Consequently, respective pressures in the conduit between the valves V4 and V6, and in a section of the conduit between the valves V6 and V7 decrease to the high vacuum region and to the ultra-high vacuum region. The valve V4 is opened to introduce the to-be-purified target gas into a section between the valves V4 and V6 (step ST10). Consequently, a pressure in this section is raised to the medium vacuum region (the pressure is measured using the pressure gauge P2 when the gas is introduced).

The getter valve GV1 is opened to enable the first getter G1 to work for reaction (step ST11). The first getter G1 has been heated to about 400±10°C in advance for reaction. After the first getter G1 is enabled to work for reaction about ten minutes, the getter valve GV1 is closed (step ST12).

Next, the getter valve GV2 is opened to enable the second getter G2 (step ST13) to work for reaction. The second getter G2 has been heated to about 400±10°C in advance for reaction. After the second getter G2 is enabled to work for reaction about ten minutes, the getter valve GV2 is closed (step ST14).

Next, the getter valve GV3 is opened to enable the third getter G3 (step ST15) to work for reaction. The third getter G3 has been heated to about 650±10°C in advance for reaction. The third getter G3 is enable to work for reaction about ten minutes and is further enabled to work for continuous reaction while being cooled about ten minutes down to room temperature, and then the getter valve GV3 is closed (step ST16). Because of the getter effect of the first to third getters G1 to G3, a pressure in the section between the valves V4 and V6 decreases to the high vacuum region or around the boundary between the high vacuum region and the ultra-high vacuum region.

After that, the valve V6 is opened to introduce the getter-processed gas into a section between the valves V6 and V7 (step ST17). Consequently, a pressure in this section is raised to the high vacuum region or around the boundary between the high vacuum region and the ultra-high vacuum region (the pressure is measured using the pressure gauge P3 when the gas is introduced). Then, the getter valve GV4 is opened to enable the fourth getter G4 (step ST18) to work for reaction. The getter valve GV4 is closed (step ST19). The fourth getter G4 is enabled to work for reaction in a heated state or at room temperature. In the embodiment, a gas obtained after the getter process performed by the fourth getter G4 is the target gas (rare gas). If a pressure in the ultra-high vacuum region is not achieved in the section between the valves V6 and V7 even after the getter process performed by the fourth getter G4, the amount of the target gas is too large. Therefore, the amount of the gas can be adjusted by closing the valve V6 and opening the valve V5 to evacuate a part of the gas using the vacuum pump VP2 (steps ST20 and ST21). Depending on the purpose of analysis and the analysis device, the obtained target gas may be directly introduced into the analysis device 11, or if further separation of the obtained target gas is needed, each rare gas may be separated from the obtained target gas by opening and closing a separation trap valve VST to enable the separation trap ST (steps ST22 and ST23) to work for reaction and the individual rare gas is introduced into the analysis device 11 (step ST24).

The term "vacuum region" as used herein is prescribed by JIS Z 8126-1:1999. Specifically, the low vacuum region means a pressure range of 100 Pa or more as prescribed by JIS. The medium vacuum region means a pressure range of less than 100 Pa to 0.1 Pa or more as prescribed by JIS. The high vacuum region means a pressure range of less than 0.1 Pa to 10⁻⁵ Pa or more as prescribed by JIS. The ultra-high vacuum region means a pressure range of less than 10⁻⁵ Pa as prescribed by JIS.

### <Getter Effect of First and Second Getters>

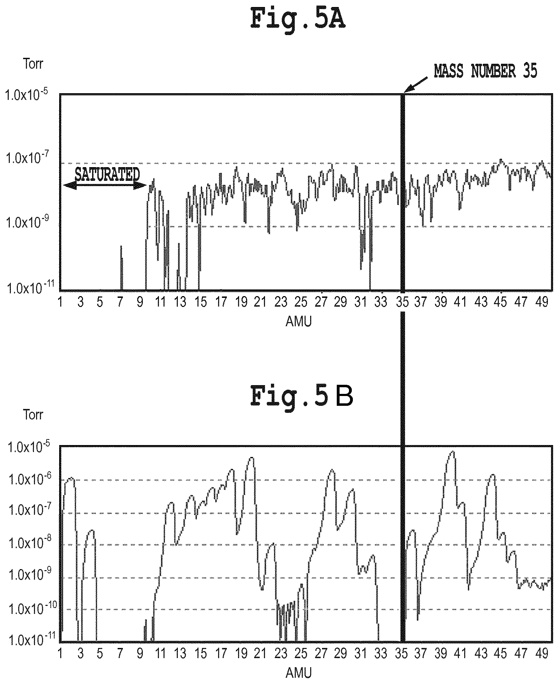

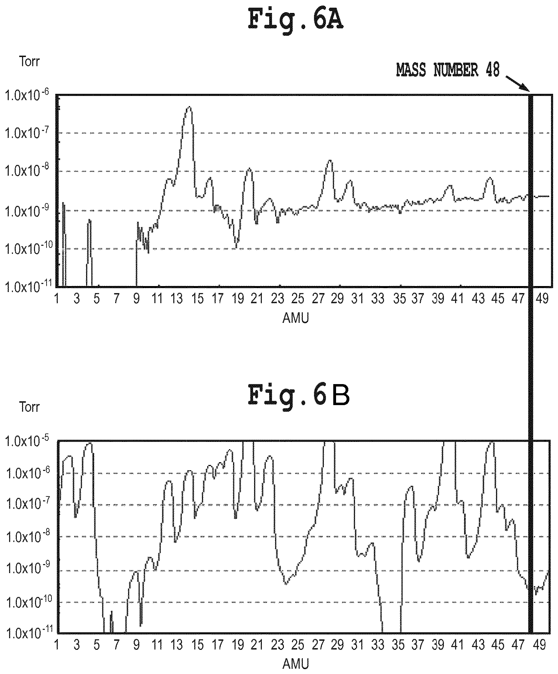

The getter effect of the first and second getters G1 and G2 will be described with reference to Figs. 5 and 6.

Fig. 5A indicates the analysis of tap water (sample water containing much chlorine) that has not been subjected to a getter process. Fig. 5B indicates the analysis of the tap water after it was subjected to the process of the first getter G1. The vertical axis represents the pressure in a logarithmic scale (torr, where 1 Torr=133.322368 Pa). The horizontal axis represents the mass number or atomic mass unit (AMU). In Fig. 5A, the analysis device was saturated by a gas at a high pressure exceeding the operation limit of the analysis device (a pressure about the medium vacuum region), and a mass spectrum was not obtained. In Fig. 5B, a pressure in the device was reduced by the effect of the getter G1, and various types of separated gases were detected for each mass number (1 AMU resolution). Among these peaks, chlorine with a mass number of 35, which corresponds to the mass number of the main chlorine isotope, chlorine-35, was not detected. By comparing Figs. 5A and 5B, it is clear that chlorine was reduced by three orders of magnitude in Fig. 5B.

Fig. 6A indicates the analysis of hot spring water (sample water containing a large amount of sulfur compounds) that has not been subjected to a getter process. Fig. 6B indicates the analysis of the hot spring water after it was subjected to the process of the second getter G2. The vertical axis represents the pressure in a logarithmic scale (torr, where 1 Torr=133.322368 Pa). The horizontal axis represents the mass number or atomic mass unit (AMU). In Fig. 6A, the analysis device was almost saturated by a gas at a pressure close to the operation limit of the analysis device (a pressure about the boundary between the medium vacuum and the high vacuum), and the mass spectrum was disturbed. Among these, sulfur monoxide with a mass number of 48, which corresponds to the mass number of sulfur monoxide obtained by bonding the main sulfur isotope, sulfur-32 and the main oxygen isotope, oxygen -16, was barely detected, and was at a pressure in the order of 10 ⁻⁷ Pa (10⁻⁹ torr). In Fig. 6B, a pressure in the device was reduced by the effect of the getter G2, and various types of separated gases were detected for each mass number (1 AMU resolution). At a mass number of 48 corresponding to the mass number of sulfur monoxide, it was located in a valley of the mass spectrum in the order of 10 ⁻⁸ Pa (10⁻¹⁰ torr). By comparing Figs. 6A and 6B, it is clear that the sulfur compound was reduced by one to two orders of magnitude in Fig. 6B.

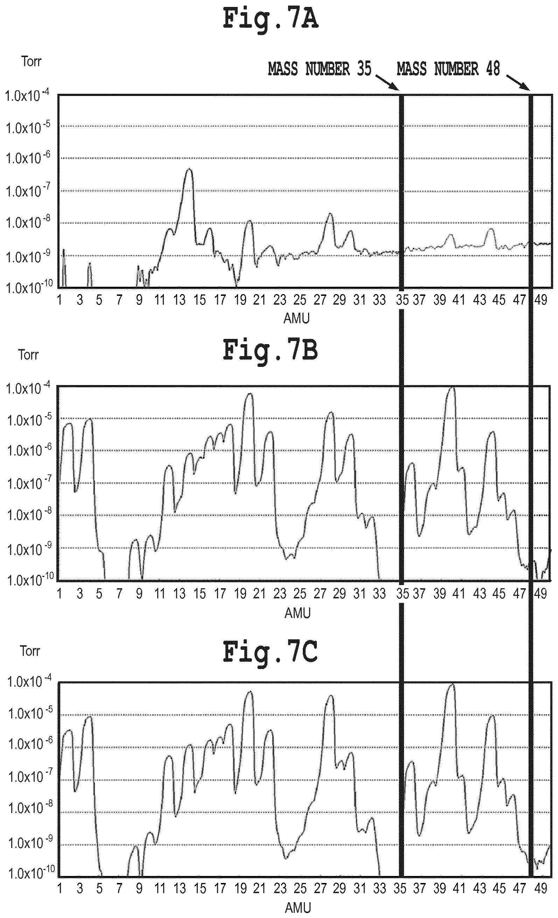

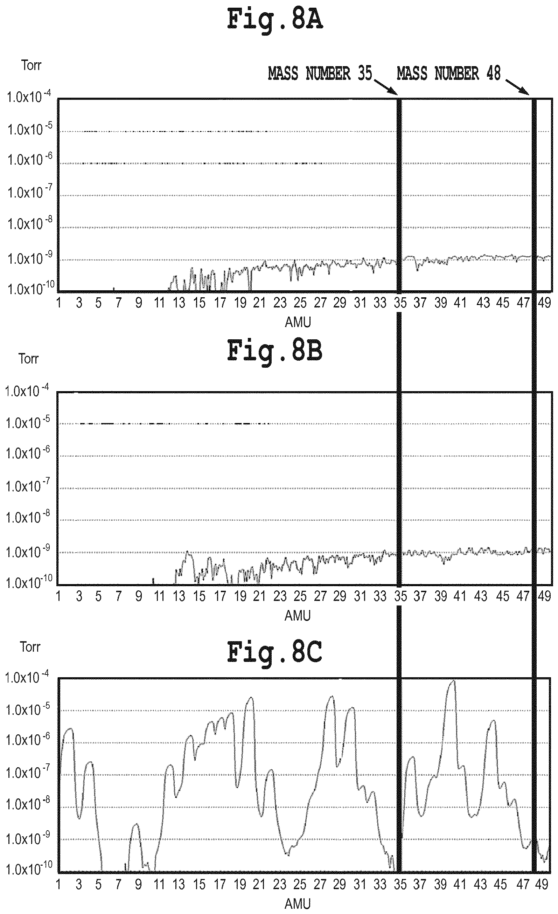

Next, the difference in getter effect due to the difference in processing order of the first and second getters G1 and G2 will be described with reference to Figs. 7 and 8. In the example of Fig. 7, the getter process was sequentially performed in the order of the first getter and then the second getter. Fig. 7A illustrates the analysis of sample water containing a halogen compound and a sulfur compound that has not been subjected to a getter process. Fig. 7B illustrates the analysis of the sample water after it was subjected to the process of the first getter. Fig. 7C illustrates the analysis of the sample water after it was subjected to the process of the second getter. The vertical axis represents the pressure in a logarithmic scale (torr, where 1 Torr=133.322368 Pa). The horizontal axis represents the mass number or atomic mass unit (AMU). As can be observed from Figs. 7A to 7C, the first and second getters each demonstrated a sufficient getter effect if the getter process was first performed by the first getter.

In the example of Fig. 8, in contrast, the getter process was sequentially performed in the order of the second getter and then the first getter. Fig. 8A illustrates the analysis of sample water containing a halogen compound and a sulfur compound that has not been subjected to a getter process. Fig. 8B illustrates the analysis of the sample water after it was subjected to the process of the second getter. Fig. 8C illustrates the analysis of the sample water after it was subjected to the process of the first getter. The vertical axis represents the pressure in a logarithmic scale (torr, where 1 Torr= 133.322368 Pa). The horizontal axis represents the mass number or atomic mass unit (AMU). As can be observed from Figs. 8A to 8C, when the getter process was first performed by the second getter, a getter effect was not achieved and a mass spectrum was not obtained even when the second getter was enabled to work for reaction. Moreover, a peak corresponding to sulfur monoxide remained at a mass number of 48 even after the subsequent getter process was performed by the first getter. Thus, it can be observed that the sulfur compound was not fully adsorbed.

Thus, it can be known from Figs. 7A to 7C and Figs. 8A to 8C that the processing order of the first and second getters is an important factor in the present invention.

While an embodiment of the present invention has been specifically described above by way of example, the present invention is not limited to such an embodiment, and it is a matter of course that modifications and variations may be made within the scope of the technical concept of the present invention. For example, the sample water is not limited to seafloor hydrothermal fluid. The preprocessing apparatus for gas analysis according to the present invention can be applied to any kind of sample water, and is particularly suitable for environmental water such as seawater and on-shore hot spring water.

### INDUSTRIAL APPLICABILITY

According to the present invention, it is possible to provide a preprocessing apparatus and a preprocessing method for gas analysis that are capable of separating and extracting a rare gas (target gas to be extracted) as a subject substance with high purity from sample water such as seafloor hydrothermal fluid in which a halogen compound, a sulfur compound, and the rare gas are dissolved.

## Claims

1. A preprocessing apparatus for gas analysis comprising:
a water trapping section (3) configured to trap water from sample water, in which a target gas to be extracted is dissolved, to extract a to-be-purified target gas; and
an adsorption section (5) configured to adsorb a non-target gas for extraction in the to-be-purified target gas, **characterized in that**:
the water trapping section (3) includes:
a first cold trap (CT1) configured to cool the sample water, which has flowed in a first trap path, at a first temperature at which the target gas is not condensed, thereby removing a liquid phase to obtain a to-be-purified target gas containing water vapor, and to establish a first vacuum environment in the first trap path, and
a second cold trap (CT2) configured to further cool the to-be-purified target gas containing water vapor, which has been purified by the first cold trap (CT1) and then has flowed in a second trap path, at a second temperature at which the water vapor is condensed but the target gas is not condensed and which is lower than the first temperature, thereby removing the water vapor to obtain the to-be-purified target gas not containing water vapor, and to establish a second vacuum environment in the second trap path such that the pressure in the second vacuum environment is lower than that in the first vacuum environment; and
the adsorption section (5) includes at least one getter that adsorbs the non-target gas for extraction from the to-be-purified target gas in a getter placement path and establishes a vacuum environment in the getter placement path such that the pressure in the getter placement path is lower than that in the water trapping section (3).

2. The preprocessing apparatus for gas analysis according to claim 1, wherein:
the adsorption section (5) includes a first adsorption sub-section provided with a first getter (G1) that adsorbs a halogen compound in the to-be-purified target gas; and a second adsorption sub-section provided with a second getter (G2) that adsorbs a sulfur compound in the to-be-purified target gas that has flowed in the getter placement path.

3. The preprocessing apparatus for gas analysis according to claim 2, wherein:
the first getter (G1) includes an iron (Fe) based getter material (GM11), a magnesium (Mg) based getter material (GM12), and a calcium (Ca) based getter material (GM13) that are arranged along an inflow path of the first getter (G1) for the to-be-purified target gas.

4. The preprocessing apparatus for gas analysis according to claim 3, wherein:
the iron based getter material (GM11) is an iron wire;
the magnesium based getter material (GM12) is a magnesium ribbon; and
the calcium based getter material (GM13) is granular calcium.

5. The preprocessing apparatus for gas analysis according to claim 3 or 4, wherein:
a weight ratio of the calcium based getter material (GM13), the magnesium based getter material (GM12), and the iron based getter material (GM11) is 1±10% : 1±10% : 0.03±10%.

6. The preprocessing apparatus for gas analysis according to claim 2, wherein:
the second getter (G2) includes a copper oxide (CuO) based getter material (GM21), an iron oxide (FeO) based getter material (GM22), a first aluminum (Al) based getter material (GM23), a vanadium (V) based getter material (GM24), a zirconium (Zr) based getter material (GM25), and a second aluminum (Al) based getter material (GM26) that are arranged along an inflow path of the second getter (G2) for the to-be-purified target gas.

7. The preprocessing apparatus for gas analysis according to claim 6, wherein:
the first aluminum based getter material (GM23) and the second aluminum based getter material (GM26) are each an aluminum foil;
the zirconium based getter material (GM25) is plate-shaped zirconium;
the vanadium based getter material (GM24) is plate-shaped vanadium;
the iron oxide based getter material (GM22) is an iron oxide wire; and
the copper oxide based getter material (GM21) is a copper oxide wire.

8. The preprocessing apparatus for gas analysis according to claim 6 or 7, wherein:
a weight ratio of the first aluminum based getter material (GM23), the zirconium based getter material (GM25), the vanadium based getter material (GM24), the second aluminum based getter material (GM26), the iron oxide based getter material (GM22), and the copper oxide based getter material (GM21) is 0.5±10% : 1±10% : 1±10% : 0.5±10% : 1±10% : 2±10%.

9. The preprocessing apparatus for gas analysis according to claim 2, wherein:
the adsorption section (5) further includes a third adsorption sub-section configured to adsorb an active gas in the to-be-purified target gas that has been subjected to an adsorption process performed by the second adsorption sub-section.

10. The preprocessing apparatus for gas analysis according to claim 9, wherein:
the adsorption section (5) further includes a fourth adsorption sub-section configured to adsorb hydrogen in the to-be-purified target gas that has been subjected to an adsorption process performed by the third adsorption sub-section.

11. A preprocessing method for gas analysis comprising the steps of:
trapping water in sample water, in which a target gas to be extracted is dissolved, to extract to-be-purified target gas; and
adsorbing a non-target gas for extraction in the to-be-purified target gas, **characterized in that**:
the step of trapping water includes:
a first trapping step of cooling the sample water, which has flowed in a first trap path, at a first temperature at which the target gas is not condensed, thereby removing a liquid phase to obtain a to-be-purified target gas that contains water vapor, and establishing a first vacuum environment in the first trap path, and
a second trapping step of further cooling the to-be-purified target gas that contains water vapor, which has been purified in the first trapping step and then has flowed in a second trap path, at a second temperature at which the water vapor is condensed but the target gas is not condensed and which is lower than the first temperature, thereby removing the water vapor to obtain a to-be-purified target gas that does not contain water vapor, and establishing a second vacuum environment in the second trap path such that the pressure in the second vacuum environment is lower than that in the first vacuum environment; and
the step of adsorbing includes:
adsorbing the non-target gas for extraction from the to-be-purified target gas in a getter placement path, and
establishing a vacuum environment in the getter placement path such that the pressure in the getter placement path is lower than that in the water trapping section (3).

## Patentansprüche

1. Vorverarbeitungsvorrichtung zur Gasanalyse, umfassend:
einen Wasserauffangabschnitt (3), der zum Auffangen von Wasser aus Probenwasser, in dem ein zu extrahierendes Zielgas gelöst ist, zum Extrahieren eines zu reinigenden Zielgases ausgelegt ist; und
einen Adsorptionsabschnitt (5), der zum Adsorbieren eines Nicht-Zielgases zur Extraktion in dem zu reinigenden Zielgas ausgelegt ist, **dadurch gekennzeichnet, dass**:
der Wasserauffangabschnitt (3) beinhaltet:
eine erste Kühlfalle (CT1), die zum Kühlen des Probenwassers, das in einen ersten Auffangpfad geflossen ist, auf eine erste Temperatur, bei der das Zielgas nicht kondensiert, ausgelegt ist, wodurch eine flüssige Phase entfernt wird, um ein Wasserdampf enthaltendes, zu reinigendes Zielgas zu erhalten, und um eine erste Vakuumumgebung in dem ersten Auffangpfad herzustellen, und
eine zweite Kühlfalle (CT2), die zum weiteren Kühlen des zu reinigenden, Wasserdampf enthaltenden Zielgases, das durch die erste Kühlfalle (CT1) gereinigt wurde und anschließend in einen zweiten Auffangpfad geflossen ist, bei einer zweiten Temperatur ausgelegt ist, bei der der Wasserdampf kondensiert ist, das Zielgas jedoch nicht kondensiert ist, und die niedriger als die erste Temperatur ist, wodurch der Wasserdampf entfernt wird, um das zu reinigende Zielgas zu erhalten, das keinen Wasserdampf enthält, und um eine zweite Vakuumumgebung in dem zweiten Auffangpfad herzustellen, sodass der Druck in der zweiten Vakuumumgebung niedriger ist als der in der ersten Vakuumumgebung; und
der Adsorptionsabschnitt (5) zumindest einen Getter beinhaltet, der das Nicht-Zielgas zur Extraktion aus dem zu reinigenden Zielgas in einem Getter-Platzierungspfad absorbiert und eine Vakuumumgebung in dem Getter-Platzierungspfad herstellt, sodass der Druck in dem Getter-Platzierungspfad niedriger ist als der in dem Wasserauffangabschnitt (3).

2. Vorverarbeitungsvorrichtung zur Gasanalyse nach Anspruch 1, wobei:
der Adsorptionsabschnitt (5) einen ersten Adsorptionsunterabschnitt beinhaltet, der mit einem ersten Getter (G1) vorgesehen ist, der eine Halogenverbindung in dem zu reinigenden Zielgas absorbiert; und einen zweiten Adsorptionsunterabschnitt, der mit einem zweiten Getter (G2) vorgesehen ist, der eine Schwefelverbindung in dem zu reinigenden Zielgas absorbiert, das in den Getter-Platzierungspfad geflossen ist.

3. Vorverarbeitungsvorrichtung zur Gasanalyse nach Anspruch 2, wobei:
der erste Getter (G1) ein auf Eisen (Fe) basierendes GetterMaterial (GM11), ein auf Magnesium (Mg) basierendes GetterMaterial (GM12) und ein auf Kalzium (Ca) basierendes GetterMaterial (GM13) beinhaltet, die entlang eines Zuflussweges des ersten Getters (G1) für das zu reinigende Zielgas angeordnet sind.

4. Vorverarbeitungsvorrichtung zur Gasanalyse nach Anspruch 3, wobei:
das auf Eisen basierende Gettermaterial (GM11) ein Eisendraht ist;
das auf Magnesium basierende Gettermaterial (GM12) ein Magnesiumband ist; und
das auf Kalzium basierende Gettermaterial (GM13) ein granulares Kalzium ist.

5. Vorverarbeitungsvorrichtung zur Gasanalyse nach Anspruch 3 oder 4, wobei:
ein Gewichtsverhältnis des auf Kalzium basierenden Gettermaterials (GM13), des auf Magnesium basierenden Gettermaterials (GM12) und des auf Eisen basierenden Gettermaterials (GM11) 1±10 % : 1±10 % : 0,03±10 % beträgt.

6. Vorverarbeitungsvorrichtung zur Gasanalyse nach Anspruch 2, wobei:
der zweite Getter (G2) ein auf Kupferoxid (CuO) basierendes Gettermaterial (GM21), ein auf Eisenoxid (FeO) basierendes Gettermaterial (GM22), ein erstes auf Aluminium (Al) basierendes Gettermaterial (GM23), ein auf Vanadium (V) basierendes Gettermaterial (GM24), ein auf Zirkonium (Zr) basierendes Gettermaterial (GM25) und ein zweites auf Aluminium (Al) basierendes Gettermaterial (GM26) beinhaltet, die entlang eines Zuflussweges des zweiten Getters (G2) für das zu reinigende Zielgas angeordnet sind.

7. Vorverarbeitungsvorrichtung zur Gasanalyse nach Anspruch 6, wobei:
das erste auf Aluminium basierende Gettermaterial (GM23) und das zweite auf Aluminium basierende Gettermaterial (GM26) jeweils eine Aluminiumfolie sind;
das auf Zirkonium basierende Gettermaterial (GM25) plattenförmiges Zirkonium ist;
das auf Vanadium basierende Gettermaterial (GM24) plattenförmiges Vanadium ist;
das auf Eisenoxid basierende Gettermaterial (GM22) ein Eisenoxiddraht ist; und
das auf Kupferoxid basierende Gettermaterial (GM21) ein Kupferoxiddraht ist.

8. Vorverarbeitungsvorrichtung zur Gasanalyse nach Anspruch 6 oder 7, wobei:
ein Gewichtsverhältnis des ersten auf Aluminium basierenden Gettermaterials (GM23), des auf Zirkonium basierenden Gettermaterials (GM25), des auf Vanadium basierenden Gettermaterials (GM24), des zweiten auf Aluminium basierenden Gettermaterials (GM26), des auf Eisenoxid basierenden Gettermaterials (GM22) und des auf Kupferoxid basierenden Gettermaterials (GM21) 0,5±10 % : 1±10 % : 1±10 % : 0,5±10 % : 1±10 % : 2±10 % beträgt.

9. Vorverarbeitungsvorrichtung zur Gasanalyse nach Anspruch 2, wobei:
der Adsorptionsabschnitt (5) ferner einen dritten Adsorptionsunterabschnitt beinhaltet, der zum Adsorbieren eines aktiven Gases in dem zu reinigenden Zielgas ausgelegt ist, das einem von dem zweiten Adsorptionsunterabschnitt ausgeführten Adsorptionsprozess unterzogen wurde.

10. Vorverarbeitungsvorrichtung zur Gasanalyse nach Anspruch 9, wobei:
der Adsorptionsabschnitt (5) ferner einen vierten Adsorptionsunterabschnitt beinhaltet, der zum Adsorbieren von Wasserstoff in dem zu reinigenden Zielgas, das einem von dem dritten Adsorptionsunterabschnitt ausgeführten Adsorptionsprozess unterzogen wurde, ausgelegt ist.

11. Vorverarbeitungsverfahren zur Gasanalyse, umfassend die Schritte:
Auffangen von Wasser in Probenwasser, in dem ein zu extrahierendes Zielgas gelöst ist, zum Extrahieren eines zu reinigenden Zielgases; und
Adsorbieren eines Nicht-Zielgases zur Extraktion in dem zu reinigenden Zielgas, **dadurch gekennzeichnet, dass**:
der Schritt des Auffangens von Wasser beinhaltet:
einen ersten Auffangschritt des Kühlens des Probenwassers, das in einen ersten Auffangpfad geflossen ist, auf eine erste Temperatur, bei der das Zielgas nicht kondensiert ist, wodurch eine flüssige Phase entfernt wird, um ein zu reinigendes Zielgas zu erhalten, das Wasserdampf enthält, und das Herstellen einer ersten Vakuumumgebung in dem ersten Auffangpfad, und
einen zweiten Auffangschritt des weiteren Kühlens des zu reinigenden, Wasserdampf enthaltenden Zielgases, das in dem ersten Auffangschritt gereinigt wurde und anschließend in einen zweiten Auffangpfad geflossen ist, bei einer zweiten Temperatur, bei der der Wasserdampf kondensiert ist, das Zielgas jedoch nicht kondensiert ist, und die niedriger als die erste Temperatur ist, wodurch der Wasserdampf entfernt wird, um ein zu reinigendes Zielgas zu erhalten, das keinen Wasserdampf enthält, und Erzeugen einer zweiten Vakuumumgebung in dem zweiten Auffangpfad, sodass der Druck in der zweiten Vakuumumgebung niedriger ist als der in der ersten Vakuumumgebung; und
der Schritt des Adsorbierens beinhaltet:
Absorbieren des Nicht-Zielgases zur Extraktion aus dem zu reinigenden Zielgas in einem Getter-Platzierungspfad, und
Herstellen einer Vakuumumgebung in dem Getter-Platzierungspfad, sodass der Druck in dem Getter-Platzierungspfad niedriger ist als der in dem Wasserauffangabschnitt (3).

## Revendications

1. Appareil de prétraitement pour une analyse de gaz comprenant :
une section de piégeage d'eau (3) configurée pour piéger l'eau de l'eau échantillon, dans laquelle un gaz cible à extraire est dissous, pour extraire un gaz cible à purifier ; et
une section d'adsorption (5) configurée pour adsorber un gaz non cible pour une extraction dans le gaz cible à purifier, **caractérisé en ce que** :
la section de piégeage d'eau (3) inclut :
un premier piège froid (CT1) configuré pour refroidir l'eau échantillon, qui a circulé dans une première voie de piégeage, à une première température à laquelle le gaz cible n'est pas condensé, éliminant ainsi une phase liquide pour obtenir un gaz cible à purifier contenant de la vapeur d'eau, et pour établir un premier environnement sous vide dans la première voie de piégeage, et
un deuxième piège froid (CT2) configuré pour refroidir davantage le gaz cible à purifier contenant de la vapeur d'eau, qui a été purifié par le premier piège froid (CT1) et a ensuite circulé dans une deuxième voie de piégeage, à une deuxième température à laquelle la vapeur d'eau est condensée mais le gaz cible n'est pas condensé et qui est inférieure à la première température, éliminant ainsi la vapeur d'eau pour obtenir le gaz cible à purifier ne contenant pas de vapeur d'eau, et pour établir un deuxième environnement sous vide dans le deuxième chemin de piégeage de telle sorte que la pression dans le deuxième environnement sous vide est inférieure à celle dans le premier environnement sous vide ; et
la section d'adsorption (5) comporte au moins un getter qui absorbe le gaz non cible pour une extraction du gaz cible à purifier dans une voie de placement de getter et établit un environnement sous vide dans la voie de placement de getter de telle sorte que la pression dans la voie de placement de getter est inférieure à celle dans la section de piégeage d'eau (3).

2. Appareil de prétraitement pour l'analyse de gaz selon la revendication 1, dans lequel :
la section d'adsorption (5) comporte une première sous-section d'adsorption munie d'un premier getter (G1) qui absorbe un composé halogène dans le gaz cible à purifier ; et une deuxième sous-section d'adsorption munie d'un deuxième getter (G2) qui absorbe un composé de soufre dans le gaz cible à purifier qui a circulé dans la voie de placement de getter.

3. Appareil de prétraitement pour une analyse de gaz selon la revendication 2, dans lequel :
le premier getter (G1) comprend un matériau getter à base de fer (Fe) (GM11), un matériau getter à base de magnésium (Mg) (GM12) et un matériau getter à base de calcium (Ca) (GM13) qui sont agencés le long d'une voie de circulation entrante du premier getter (G1) pour le gaz cible à purifier.

4. Appareil de prétraitement pour une analyse de gaz selon la revendication 3, dans lequel :
le matériau getter à base de fer (GM11) est un fil de fer ;
le matériau getter à base de magnésium (GM12) est un ruban de magnésium ; et
le matériau getter à base de calcium (GM13) est du calcium granulaire.

5. Appareil de prétraitement pour une analyse de gaz selon la revendication 3 ou 4, dans lequel :
un rapport pondéral du matériau getter à base de calcium (GM13), du matériau getter à base de magnésium (GM12) et du matériau getter à base de fer (GM11) est de 1 ± 10 % : 1 ± 10 % : 0,03 ± 10 %.

6. Appareil de prétraitement pour une analyse de gaz selon la revendication 2, dans lequel :
le deuxième getter (G2) comporte un matériau getter à base d'oxyde de cuivre (CuO) (GM21), un matériau getter à base d'oxyde de fer (FeO) (GM22), un premier matériau getter à base d'aluminium (Al) (GM23), un matériau getter à base de vanadium (V) (GM24), un matériau getter à base de zirconium (Zr) (GM25), et un deuxième matériau getter à base d'aluminium (Al) (GM26) qui sont disposés le long d'une voie de circulation entrante du deuxième getter (G2) pour le gaz cible à purifier.

7. Appareil de prétraitement pour une analyse de gaz selon la revendication 6, dans lequel :
le premier matériau getter à base d'aluminium (GM23) et le deuxième matériau getter à base d'aluminium (GM26) sont chacun une feuille d'aluminium ;
le matériau getter à base de zirconium (GM25) est du zirconium en forme de plaque ;
le matériau getter à base de vanadium (GM24) est du vanadium en forme de plaque ;
le matériau getter à base d'oxyde de fer (GM22) est un fil d'oxyde de fer ; et
le matériau getter à base d'oxyde de cuivre (GM21) est un fil d'oxyde de cuivre.

8. Appareil de prétraitement pour une analyse de gaz selon la revendication 6 ou 7, dans lequel :
un rapport pondéral du premier matériau getter à base d'aluminium (GM23), du matériau getter à base de zirconium (GM25), du matériau getter à base de vanadium (GM24), du deuxième matériau getter à base d'aluminium (GM26), du matériau getter à base d'oxyde de fer (GM22) et du matériau getter à base d'oxyde de cuivre (GM21) est de 0,5 ± 10 % : 1 ± 10 % : 1 ± 10 % : 0,5 ± 10 % : 1 ± 10 % : 2 ± 10 %.

9. Appareil de prétraitement pour une analyse de gaz selon la revendication 2, dans lequel :
la section d'adsorption (5) comporte en outre une troisième sous-section d'adsorption configurée pour adsorber un gaz actif dans le gaz cible à purifier qui a été soumis à un processus d'adsorption réalisé par la deuxième sous-section d'adsorption.

10. Appareil de prétraitement pour une analyse de gaz selon la revendication 9, dans lequel :
la section d'adsorption (5) comporte en outre une quatrième sous-section d'adsorption configurée pour adsorber l'hydrogène dans le gaz cible à purifier qui a été soumis à un processus d'adsorption réalisé par la troisième sous-section d'adsorption.

11. Procédé de prétraitement pour une analyse de gaz comprenant les étapes :
de piégeage de l'eau dans l'eau échantillon, dans lequel un gaz cible à extraire est dissous, pour extraire le gaz cible à purifier ; et
d'adsorption d'un gaz non cible pour l'extraction dans le gaz cible à purifier, **caractérisé en ce que** :
l'étape de piégeage de l'eau comporte :
une première étape de piégeage consistant à refroidir l'eau échantillon, qui a circulé dans une première voie de piégeage, à une première température à laquelle le gaz cible n'est pas condensé, éliminant ainsi une phase liquide pour obtenir un gaz cible à purifier qui contient de la vapeur d'eau, et établir un premier environnement sous vide dans la première voie de piégeage, et
une deuxième étape de piégeage consistant à refroidir davantage le gaz cible à purifier qui contient de la vapeur d'eau, qui a été purifié dans la première étape de piégeage et a ensuite circulé dans une deuxième voie de piégeage, à une deuxième température à laquelle la vapeur d'eau est condensée mais le gaz cible n'est pas condensé et qui est inférieure à la première température, éliminant ainsi la vapeur d'eau pour obtenir un gaz cible à purifier qui ne contient pas de vapeur d'eau, et l'établissement d'un deuxième environnement sous vide dans la deuxième voie de piégeage de telle sorte que la pression dans le deuxième environnement sous vide est inférieure à celle dans le premier environnement sous vide ; et
l'étape d'adsorption comporte :
l'absorption du gaz non cible pour l'extraction du gaz cible à purifier dans une voie de placement de getter, et
l'établissement d'un environnement sous vide dans la voie de placement de getter de telle sorte que la pression dans la voie de placement de getter est inférieure à celle dans la section de piégeage d'eau (3).
